Europäisches Patentamt

European Patent Office

Office européen des brevets

(11) Publication number: **0 119 767**
**B1**

# EUROPEAN PATENT SPECIFICATION

(45) Date of publication of patent specification: **22.11.90**

(21) Application number: **84301154.5**

(22) Date of filing: **22.02.84**

(51) Int. Cl.⁵: **G 01 N 33/535,**
**G 01 N 33/542**

(54) Method of measuring ligands.

(30) Priority: **11.03.83 JP 38975/83**
**29.03.83 JP 51494/83**
**29.03.83 JP 51495/83**

(43) Date of publication of application:
**26.09.84 Bulletin 84/39**

(45) Publication of the grant of the patent:
**22.11.90 Bulletin 90/47**

(84) Designated Contracting States:
**CH DE FR GB IT LI NL**

(56) References cited:
**EP-A-0 122 833**
**US-A-4 160 645**
**US-A-4 341 866**

(73) Proprietor: **FUJIREBIO KABUSHIKI KAISHA also
trading as FUJIREBIO INC.**
**6-7, Shimoochiai 4-chome Shinjuku-ku
Tokyo 161 (JP)**

(72) Inventor: **Ashihara, Yoshihiro**
**2-402, Fuchudanchi 28-1 Harumicho 1-chome
Fuchu-shi Tokyo (JP)**
Inventor: **Suzuki, Hiromasa**
**36-12, Shimizucho
Itabashi-ku Tokyo (JP)**
Inventor: **Kasahara, Yasushi**
**310, 4-4, Nagayama 3-chome
Tama-shi Tokyo (JP)**

(74) Representative: **Silverman, Warren et al
HASELTINE LAKE & CO. Hazlitt House
28 Southampton Buildings Chancery Lane
London WC2A 1AT (GB)**

Courier Press, Leamington Spa, England.

**Description**

This invention relates to a method of determining a biological ligand which may be, for example, a medicinal substance or a compound resulting from one of various diseases and to be found in a body fluid, such as blood serum or urine.

Analyses of such trace constituents are useful as means for diagnosing various diseases and determining treatment to be given, and, accordingly, they are utilised as clinical tests. However, body fluids such as blood serum and urine contain a variety of constituents, and some of them are similar to each other in their molecular weights, physiological activities, or molecular structures. Thus, high specificity and high sensitivity are required of methods to determine such trace constituents. Moreover, these methods also need to be simple, since they are often employed as frequently repeated routine tests.

Immunoassay as a testing method utilises high affinity between an antigen and an antibody as well as the high specificity of an antibody which discriminates an antigenic determinant. Immunoassay testing methods hitherto used which take advantage of these features include radioimmunoassay, enzyme immunoassay and a method utilising hemagglutination.

The sensitivity of the radioimmunoassay method is high. However, this method uses radioisotopes which are harmful to human beings, and accordingly, the place where the radioimmunoassay is carried out and the amount of radioisotope to be used are severely regulated, and special facilities are needed. Such a problem does not, however, exist with enzyme immunoassay. However, as in radioimmunoassay, there is a need to carry out separation of a free labelled-substance from a bound labelled-substance. These separation procedures are complicated, and there are difficulties in carrying them out and significant scope for measurement errors. When carrying out the method utilising hemagglutination, such a separation procedure is not necessary. However, the sensitivity of this method is lower than in either of the above two methods, and the measuring of a very small amount of substance, of the order of nanograms and picograms, is difficult.

One immunoassay technique which claims to operate effectively and which does not include a separation step as aforesaid forms the subject of EP—A—0034050. The method of this specification comprises forming a mixture of:

a) sample;

b) a substance bearing a non-radioisotopic label, which substance is the same as the antigen (Ag) under assay or is so closely similar as to be bindable by an antibody against the Ag;

c) an antibody against the Ag;

and detecting the amount of label activity in the mixture and therefrom determining the amount of Ag, in which method component (c) is a mixed binding reagent which comprises at least one first site selectively bindable to the Ag, and at least one second site selectively bindable to the label, the first and second sites being spaced apart so that a single molecule of labelled substance cannot simultaneously become bound to both a first and a second site, and wherein the activity of the label is changed upon binding to the second sites.

According to the present invention there is provided a method of determining a biological ligand which comprises, bringing about contact in an aqueous medium between substances (a) to (c) of which substance (a) is a sample considered to contain a ligand to be measured, the ligand being of macromolecular size or a hapten, substance (b) is an enzyme or a bound complex of an enzyme and a water-soluble macromolecular substance, and substance (c) is either a bound complex of an antibody against said ligand and an antibody against said enzyme or a bound complex of an antibody against said ligand, an antibody against said enzyme and a water-soluble macromolecular substance, one of substances (a), (b) and (c) being of macromolecular size with a substance (b) or (c), in the absence of a substance (a) of macromolecular size, achieving such macromolecular size as a result of its being a said bound complex with a water-soluble macromolecular substance having a molecular weight greater than 100,000; determining the residual activity of said enzyme and utilising the result obtained as a measure of the ligand or an indication of the absence thereof.

According to the present invention there also is provided a method of determining a biological ligand which comprises bringing about contact in an aqueous medium between substances (a) to (d) of which substance (a) is a sample considered to contain the ligand to be measured, the ligand being of macromolecular size or a hapten, substance (b) is an enzyme or a bound complex of an enzyme and a water-soluble macromolecular substance, substance (c) is either a bound complex of an antibody against said ligand and an antibody against said enzyme or a bound complex of an antibody against said ligand, an antibody against said enzyme and a water-soluble macromolecular substance and substance (d) is a bound complex of a said ligand and a water-soluble macromolecular substance which has a molecular weight of more than 100,000 daltons and/or a polymerized form of said ligand; determining the residual activity of said enzyme and utilising the result obtained as a measure of the ligand or an indication of the absence thereof.

Furthermore, the present invention encompasses also a method of determining a biological ligand which comprises bringing about contact in an aqueous medium between substances (a) to (c) and (e) of which substance (a) is a sample considered to contain the ligand to be measured, the ligand being of macromolecular size or a hapten, substance (b) is an enzyme or a bound complex of an enzyme and a

2

water-soluble macromolecular substance, substance (c) is either a bound complex of an antibody against said ligand and an antibody against said enzyme or a bound complex of an antibody against said ligand, an antibody against said enzyme and a water-soluble macromolecular substance and substance (e) is a second antibody which is an antibody against said antibody against said ligand or a bound complex of a said second antibody against said antibody against said ligand and a water-soluble macromolecular substance and/or is a second antibody which is an antibody against said antibody against said enzyme or a bound complex of a said antibody against said antibody against said enzyme and a water-soluble macromolecular substance; determining the residual activity of said enzyme and utilising the result obtained as a measure of the ligand or an indication of the absence thereof.

This invention is based on the discovery that when an antibody against a particular ligand to be measured and an antibody against a particular enzyme are present as a bound complex and contacted with the ligand to be measured and the enzyme, and steric hindrance can occur (see hereinafter), the enzyme activity varies according to the amount of the ligand. By utilising this raction, the ligand can easily be measured with high sensitivity,

A ligand in the biological or medical field may be described as a substance having one or more antigen determinants, and such ligands include hormones derived from various endocrine glands, and exemplified by insulin, TSH and thyroglobulin; plasma proteins, for example immunoglobulin, albumen and ferritin and viral antigens, for example HB antigen, bacteria, α-fetoprotein, and carcinoembryonic antigens, all of which ligands are of macromolecular size. The method of this invention works particularly well with ligands having a molecular weight of more than 100,000 daltons in enabling sufficient variation of enzyme activity to be accommodated. However, lower molecular haptens may also be measured easily by the method of this invention by using additionally a macromolecular ligand or a polymerised ligand, as will be described later. Such haptens include medicinal substances such as digoxin, theophylline, phenobarbital, phenytoin, penicillin and amikacin, and hormones such as prostaglandin, testosterone, progesterone and thyroxin.

It is preferable to use an enzyme whose activity is easily measured. Moreover, it is necessary to obtain the antibody of the enzyme. As with most enzymes, the antibodies can easily be produced by the injection of the enzyme into the body of an animal. In the case of enzymes derived from an animal, the antibodies can also be produced by the injection into the body of another animal. Examples of such enzymes include glucose-6-phosphate dehydrogenase, hexokinase, α-amylase, malate dehydrogenase, alkaline phosphatase, peroxidase, β-galactosidase, creatine kinase, ribonuclease, and penicillinase.

When an enzyme is allowed to react with an antibody bound complex as will be described later, the enzyme activity does not appreciably vary, even when, as is preferably the case, a macromolecular substance is bound to the enzyme prior to use. The bound complex of an enzyme and a macromolecular substance is termed hereinafter a macromolecular enzyme. The macromolecular substances used are water-soluble, and their molecular weights are preferably greater than 100,000 daltons. Such a macromolecular substance is able to impart its water solubility to its combination with an enzyme. Such a bound complex may be produced by first binding a relatively low molecular weight substance such as bovine serum albumen to an enzyme, and thereafter the bound bovine serum albumen is polymerized with free bovine serum albumen molecules by self-polymerisation. Other types of macromolecular substance which may be used include polysaccharides and their derivatives, such as soluble dextran, carboxymethyl dextran, dextran induced amino group and amylose, proteins such as gelatin, hemocyanin and ferritin, and polyethylene glycol.

This binding with the macromolecular substance need not only take place with the enzyme, but may be applied to the antibody bound complex as will be described later. Moreover, both the enzyme and the antibody bound complex may be bound to a macromolecular substance.

The binding method used will usually depend on the functional groups of both substances to be bound together. Use may be made of such functional groups as amino groups, carboxyl groups, hydroxyl groups, thiol groups, imidazole groups and phenyl groups. Thus, when forming a bond between two amino groups, many methods such as the diisocyanate method, the glutaraldehyde method, the difluorobenzene method, and the benzoquinone method, are available. When binding an amino group and a carboxyl group, one can use the peptide-binding method for joining a carboxyl group to succinimido ester, the carbodiimide method and the Woodward reagent method. The periodate oxidation method (Nakane method) can be used where bridge formation between an amino group and a sugar chain is to be formed. When a thiol group of one reagent molecule is to be involved in bond formation, a carboxyl group of the other reagent molecule is first converted to succinimido ester, and this ester group is then allowed to react with cysteine to introduce a free thiol group into the other reagent molecule and the thiol groups of the respective molecules are bound by using a thiol-reactive bifunctional cross-linking reagent such as phenylene-bismaleimide. Phenyl groups may be linked using the diazotization method and the alkylation method. These and other binding methods may be selected as appropriate from the various methods described in "Method in Immunology and Immunochemistry" (C. A. Williams et al. 1976, Academic Press N.Y.) and "Koso Meneki Sokutei-ho" (E. Ishikawa et al. 1978, Igaku-shoin (Japan)). The molar ratio of substances to be bound is not limitd to 1:1, and a suitable ratio is selected. After the binding reaction, the macromolecular enzyme produced or bound complex of macromolecule and antibodies produced is generally purified by gel filtration, ion-exchange chromatography and affinity chromatography, and lyophilised, if necessary.

The antibody against the ligand to be measured (hereinafter referred to as "antiligand antibody") and the antibody against the enzyme (hereinafter referred to as "antienzyme antibody") may be produced by following conventional methods of producing an antibody. For example, the ligand or the enzyme is injected once or several times subcutaneously into the back, foot pad or femoral muscle of a warm-blooded animal, such as rabbit, goat, horse, guinea-pig or chicken, in an amount of 0.3 to 2 mg per kg together with an adjuvant, and the antibody is then produced in the body fluid of the animal. These antibodies are not limited to immunoglobulins such as IgG, IgM and IgA, and include their digestion products with pepsin, such as $F(ab')_2$, Fab' and Fab. Insofar as the production of the antienzyme antibody is concerned, some antibodies entirely inhibit enzyme activity, some antibodies partially inhibit, and the remaining do not inhibit. However any antienzyme antibody can be used in carrying the method of the invention. The antibody may be purified by a conventional isolation method for isolating immunoglobulin from serum, such as precipitation using ammonium sulphate, ion-exchange chromatography, gel filtration and affinity chromatography.

The antibodies used in a bound complex may also be produced as monoclonal antibodies. In this case, the ligand or enzyme is injected several times into the abdominal cavity of a mouse together with an adjuvant, and its spleen is excised. A spleen cell is fused with a mouse myeloma cell by a conventional method such as by using polyethylene glycol. The hybridoma thus obtained is cultured and cloned, and a cell capable of producing the antibody in question is obtained. This cell is injected into the abdominal cavity of a mouse, and multiplied. Then, ascites are collected, and the antibody is separated from the ascites.

The method used for binding the antiligand antibody and the antienzyme antibody may be selected from the binding methods suitable for binding mutual proteins among the methods for binding an enzyme and a macromolecular substance previously described herein. Often applicable are the glutaraldehyde method, the periodate oxidation method, the maleimide method, the diisocyanate method, the benzoquinone method, and the carbodiimide method. Also to be considered are the SPDP method for binding an $NH_2$ group and an SH group, a method using a lectin such as protein A which is able to bind to the sugar chain of IgG, and the rearrangement of SH groups of two kinds of $F(ab')_2$ in the presence of a reducing agent. The antibody bound complex does not have to be merely the bound complex of one molecule of the antiligand antibody to one molecule of the antienzyme antibody. There may be several molecules of each type bound to each other and the molecules may even be polymerised. The complexing ratio of each molecule is therefore not limited to 1:1.

As previously described, the antibody bound complex may be bound to a macromolecular substance. In this case, the macromolecular substance may be selected from the foregoing macromolecular substances, and the binding method used for binding it to the antibody bound complex may also be selected from the foregoing methods. The binding with the macromolecular substance may be carried out by binding one or both antibodies with the macromolecular substance prior to the binding of the antibodies, or it may be carried out after the binding of the antibodies.

The antibody bound complex and the bound complex of the antibody bound complex and a macromolecular substance (hereinafter referred to as "macromolecular antibody bound complex") are generally purified by gel filtration, ion-exchange chromatography using a cation-exchange resin or an anion-exchange resin, and affinity chromatography, and then lyophilised, if necessary.

In order to enhance accommodation of variation in the enzyme activity and, in particular, to increase enzyme sensitivity, a second antibody against the antibody against the ligand to be measured (hereinafter referred to as "second antibody of the antiligand antibody") or a second antibody against the antibody against the enzyme (hereinafter referred to as "second antibody of the antienzyme antibody") is preferably contacted with the antibody bound complex or the macromolecular antibody bound complex.

These second antibodies employed may be produced by the same method as that described above for the antiligand antibody and the antienzyme antibody. These second antibodies are also not limited to immunoglobulins, and include the pepsin digestion products such as $F(ab')_2$, $F(ab')$ and Fab and may be monoclonal antibodies. Moreover, they may be applied irrespective of their enzyme inhibitory activities. They may also be bound to a water-soluble macromolecular substance having a molecular weight of more than 100,000 daltons prior to use. In this case, the macromolecular substance may be one of those previously described herein.

An alternative method of accommodating variation of enzyme activity is to use as a further component of the assay system a bound complex of the ligand to be measured and a water-soluble macromolecular substance (hereinafter referred to as "macromolecular ligand") or the ligand to be measured in a polymerised form (hereinafter referred to as "polymerised ligand"). The bound complex or polymerised ligand is contacted with the antibody bound complex (or macromolecular antibody bound complex) and it is to the result of this contacting that the sample to be determined and the enzyme or enzyme bound complex is added. When using a macromolecular ligand or the polymerised ligand, a lower molecular ligand, for example, having a molecular weight of less than 100,00 daltons can easily be measured. Indeed the ligand can then even be a hapten as described above.

The macromolecular substance and its binding method to the ligand are essentially as previously described herein. When the ligand is polymerised, the polymerisation method used is preferably selected from the binding method for a macromolecular substance previously described herein. For example, it may be carried out by using a divalent cross-linking agent such as carbodiimide and glutaraldehyde.

After the formation thereof, the macromolecular ligand and the polymerised ligand are usually to be purified by using gel filtration, ion-exchange chromatography and affinity chromatography, and lyophilised, if necessary.

In carrying out the method of this invention, as such, the sample to be determined and the enzyme or the macromolecular enzyme are brought into contact with the antibody bound complex or the macromolecular antibody bound complex in an aqueous solution. The aqueous solution is preferably kept at 20 to 45°C at pH to 8.5. In order to maintain the pH constant, a buffer solution such as a phosphate buffer solution and an acetate buffer solution may be added thereto. The amount of the enzyme or macromolecular enzyme and of the antibody bound complex or the macromolecular antibody bound complex will vary according to their identity, the kind of ligand to be determined and the contacting conditions, and accordingly, amounts to be used are preferably determined by a preliminary test. The contacting time for the contacting of the antibody bound complex or the macromolecular antibody bound complex with the ligand and the enzyme or the macromolecular enzyme should be sufficient for reaction to take place to an adequate extent. Generally, reaction for 20 to 60 minutes at 37°C is preferable.

Moreover, as previously mentioned, one or more of a second antibody of the antiligand antibody, a second antibody of the antienzyme antibody, a macromolecular ligand or a polymerised ligand may be added. The amount to be used is decided by the variation in enzyme activity to be accommodated and will vary from substance to substance and, accordingly, is preferably determined by a preliminary test. The contacting conditions will usually be the same as described above.

No contacting order of the ligand, polymerised ligand or macromolecular ligand, the enzyme or the macromolecular enzyme and the second antibody of the antiligand antibody and/or the second antibody of the antienzyme antibody, when employed, is critical, and for example, all of them may be added at once to the antibody bound complex.

When carrying out the method of this invention, at least one constituent and preferably each constituent is a macromolecule of suitable size, and this requirement is satisfied if for example, a macromolecular substance and second antibody are employed which are appropriate to the ligand, the enzyme and the antibody bound complex used. Macromolecular substances in general enable steric hindrance to be utilised to advantage. This is of particular advantage with low molecular weight ligands when macromolecular ligands are polymerised, ligands provide the steric hindrance which may be lacking from the ligand being determined and possibly the enzyme and antibody bound complex components. For this purpose, the macromolecular ligand or the polymerised ligand and the ligand to be measured competitively react with the antibody combination, and the macromolecular ligand or polymerised enzyme bound to the antibody combination inhibits the binding of the enzyme with the antibody combination by it steric hindrance. The amount of the macromolecular ligand or the polymerised ligand which is bound will vary according to the amount of the ligand to be measured, and the amount of the enzyme which is bound will vary, in turn, according to such binding amounts. When steric hindrance is to be provided through the enzyme or antibody bound complex being part of a bound complex with a water-soluble macromolecular substance, the macromolecular substance should have essentially a molecular weight of at least 100,000 daltons.

After carrying out the contacting, the activity of the enzyme is measured. The measurement is carried out according to a method appropriate to the enzyme concerned. For example, when glucose-6-phosphate dehydrogenase is employed, a substrate solution containing glucose-6-phosphoric acid and $NADP^+$ is added to the reaction solution, and the yield of NADPH is determined by measuring the absorbance at 340 nm. When hexokinase is employed, a substrate solution containing glucose, ATP, $NADP^+$ and glucose-6-phosphate dehydrogenase is added to the reaction solution, and the yield of NADPH is measured in like manner.

In summary, the method of the invention enables a ligand to be detected and determined in high sensitivity and in high specificity. The operation of this method is simple, and a ligand can be easily and inexpensively determined.

The following examples illustrate this invention:

## Example 1

i) Preparation of Anti-Human- — Fetoprotein Goat Fab' — Antihexokinase Guinea-pig Fab' bound complex

A goat was immunised with human α-fetoprotein (AFP) in conventional manner, and anti-AFP goat antiserum was obtained. This antiserum was purified using an AFP-Sepharose 4B affinity column, and dried pure anti-AFP goat specific IgG was obtained.

0.2 mg of pepsin (made by Sigma Co.) was added to 20 mg of this pure specific IgG, and digested at 37°C overnight in 0.1M sodium acetate buffer solution of pH 4.2. The digestion product was adjusted to pH 7.8 by using 1N NaOH. The digestion product was then passed through a Sephadex® G—100 column, and the fraction containing 11 mg of F(ab')$_2$ was collected by measuring the absorbance of effluent at 280 nm.

5 mg of the anti-AFP goat F(ab')$_2$ thus obtained were dissolved in 2 mol of 0.1M phosphate buffer solution of pH 6.0 containing 1 mM EDTA, and 0.2 ml of 0.1M 2-mercaptoethylamine solution was added to this solution. The mixed solution obtained was allowed to react at 37°C for 90 minutes. The reaction solution was then passed through a column of Sephadex® G—25 (1 cm × 40 cm), and gel filtration was carried out. The void fractions were collected as the anti-AFP goat Fab' fraction. 4 mg of N,N'-(1,2-phenylene) bismaleimide were dissolved in 0.5 ml of acetone, and the volume of the solution obtained was adjusted to 10 ml by adding 0.1M sodium acetate buffer solution of pH 5.0. 1 ml of the diluted solution thus obtained was added to the above anti-AFP goat Fab' fraction, and allowed to react at 30°C for 20 minutes. This reaction mixture was passed through a column of Sephadex® G—25 (1 cm × 40 cm), and gel filtration was carried out. The void fractions were collected, and concentrated at 4°C by using polyethylene glycol 20,000. An anti-AFP goat Fab'-maleimide bound complex thus obtained.

Parallel with the aforesaid procedure, hexokinase (HK) was injected into a guinea-pig in the conventional manner, and anti-HK guinea-pig specific IgG was obtained. This specific IgC was purified by using a HK-Sepharose 4B affinity column, and pure anti-HK guinea-pig specific IgG was obtained. 20 mg of this pure speciific IgG were treated in the same manner as the case of the foregoing anti-AFP goat specific IgG, and a fraction containing 9.8 mg of anti-HK guinea-pig F(ab')₂ was obtained.

In the same manner as with the foregoing anti-AFP goat F(ab')₂, 5 mg of this F(ab')₂ were reduced by 2-mercaptoethylamine and purified by gel filtration using Sephadex® G—25 to obtain anti-HK guinea-pig Fab'.

The anti-HK guinea-pig Fab' thus obtained was mixed with the above anti-AFP goat Fab'-maleimide bound complex. The mixture was adjusted to pH 6.8 and allowed to react at 4°C for 24 hours. The reaction mixture was concentrated by using polyethylene glycol 20,000, and gel filtration was carried out by using a Sephadex® G—100 column (1 cm × 100 cm). The fractions corresponding to F(ab')₂ were collected, and 8 mg of anti-AFP goat Fab'-anti-HK guinea-pig Fab' bound complex were obtained.

ii) Preparation of Macromolecular Hexokinase

50 mg of rabbit serum albumen were dissolved in 2 ml $H_2O$, and the pH of the solution was adjusted to 6.0. 5 mg of 1-ethyl-3-(3-dimethylaminopropyl) carbodiimide hydrochloride (EDC) were added to this solution, and allowed to react at ambient temperature for 1 hour while the pH was kept at 6.0. The reaction mixture obtained was centrifuged, and the supernatant was introduced into a Sephacryl® S—300 column (1 cm × 100 cm), and gel filtration was carried out. The void fractions were collected, and concentrated by using polyethylene glycol 20,000 to obtain a macromolecular rabbit serum albumen. 10 mg of this macromolecular rabbit serum albumen were dissolved in 1 ml of $H_2O$, and adjusted to pH 6.0. 2 mg of HK and 10 mg of EDC were added to this solution, and allowed to react at ambient temperature for 30 minutes while the pH was kept at 6.0. The reaction mixture obtained was centrifuged, and the supernatant was purified by gel filtration using a Sepharose® 6B column. The fractions having HK activity were collected, and combined to provide the required macromolecular HK.

iii) Measurement of Human α-Fetoprotein

10 μg/ml samples of human α-fetoprotein (AFP) were diluted twice by twice, and 10 μl samples of 2n dilution series were prepared. A 20 μl sample of 20 mM phosphate buffered saline solution pH 7.0 containing 40 μg of the aforesaid anti-AFP goat Fab'-anti-HK guinea-pig Fab' bound complex was added to each diluted solution, and a 20 l sample of the macromolecular HK solution whose relative activity ($\Delta OD_{340nm}$/minute) was 0.06 was further added. The mixtures obtained were allowed to stand at ambient temperature for 30 minutes.

A 1 ml sample of 50 mM tris-HCl buffer solution pH 8.0 containing 0.1M D-glucose, 0.5 mM ATP, 0.2 mM NADP, 13.3 mM $MgCl_2$ and 3 IU/ml G6PDH was added to each mixture sample as substrate, and mixed well therewith. The variation of the absorbance at 340 nm was measured for each mixture, and the following results were obtained.

TABLE 1

| AFP (ng) | ($\Delta OD_{340nm}$/min) × 1000 |
|---|---|
| 0 | 20.0 |
| 68 | 17.5 |
| 135 | 16.3 |
| 270 | 15.2 |
| 540 | 12.4 |
| 1080 | 10.5 |

The same measurements were then repeated, but using 5 human serum samples of 50 μl each, and the AFP concentration of each sample was determined after plotting the results of Table 1 as a calibration

6

curve. In a comparative experiement, the AFP concentrations of the same sera were also measured by the radioimmunoassay (RIA) method.

The results obtained are shown in Table 2.

TABLE 2

| | AFP | |
|---|---|---|
| Serum | The Method of the Invention | RIA Method |
| A1 | 200 ng | 192 ng |
| A2 | 523 ng | 545 ng |
| A3 | 421 ng | 401 ng |
| A4 | 823 ng | 811 ng |
| A5 | 495 ng | 502 ng |

Example 2

i) Preparation of Anti-Human IgG Guinea-pig IgG-Anti-Glucose-6-Phosphate Dehydrogenase Guinea-pig IgG bound complex

5 mg of anti-human IgG guinea-pig IgG (α-hIgG) were dissolved in 1 ml of 0.1M phosphate buffer solution of pH 6.3, and 100 µl of 2 mg/ml 4-maleimidomethylcyclohexane-1-carboxylic acid succinimide ester (CHMS) dioxane solution were added to this solution. The mixed solution obtained was allowed to stand at ambient temperature for 1 hour. This solution was placed in a Sephadex® G—25 column (1 cm × 50 cm) and gel filtration was carried out by using 0.1M phosphate buffer solution of pH 6.5 containing 1 mM EDTA to remove unreacted CHMS. The solution of 4-maleimidomethylcyclohexane-1-carboxylic acid (CHM)-α-hIgG (CHM induced α-hIgG) bound complex thus obtained was concentrted to 1 ml.

5 mg of the anti-glucose-6-phosphate dehydrogenase guinea-pig IgG (α-G6PDHIgG) thus obtained were dissolved in 0.1M phosphate buffer solution of pH 7.5 containing 5 mM EDTA, and 100 µl of 9 mg/ml S-acetylmercaptosuccinic anhydride dimethyl sulphoxide solution were added. The mixture obtained was warmed at 37°C for 1 hour. Subsequently, 110 µl of 1M hydroxylamine solution of pH 7.5 were added and reaction was effected at 37°C for 30 minutes. The reaction mixture obtained was placed in a Sephadex® G—25 column, and gel filtration was carried out by using 0.1M phosphate buffer solution of pH 6.5 containing 1 mM EDTA to remove unreacted S-acetyl-mercaptosuccinic acid.

The SH induced α-G6PDHIgG thus obtained was concentrated to 1 ml, and 1 ml of the concentrated CHM induced α-hIgG solution previously described was added. The mixture obtained was allowed to react at 4°C overnight, and the reaction mixture then obtained was purified by gel filtration using Sephacryl® S—300 (1 cm × 120 cm) to obtain an α-hIgG-α-G6PDHIgG combination whose major components were present in a molar ratio of 1:1.

ii) Preparation of Macromolecular Bound Complex

The bound complex thus produced was dialysed against 0.1M carbonate buffer solution of pH 8.0. The protein concentration of the dialysate was adjusted to 5 mg/ml, and 20 mg of a succinimide ester of carboxydextran (MW = $10^7$) which had previously been prepared was added, and stirred at 37°C for 2 hours.

The reaction product thus obtained was then treated by gel filtration using Sepharose® 4B, and void fractions were collected. The fractions were concentrated, and the desired α-hIgG-α-G6PDHIgG-dextran bound complex (G-H-D complex) was obtained.

iii) Measurement of Human IgG

50 µl samples of the G-H-D complex were each added to 50 µl samples of human IgG solution having various known IgG concentrations, and the mixtures obtained were warmed at 37°C for 1 hour. 100 µl of glucose-6-phosphate dehydrogenase (G6PDH) were added to each mixture, and allowed to react at 37°C for 20 minutes. 1.0 ml of substrate solution of pH 8.5 containing 0.5 mM glucose-6-phosphoric acid, 1.3 mM NADP, 0.1M glycylglycine and 20 mM $MgCl_2$ was added to the reaction mixture, and the variation of absorbance at 340 nm at 30°C was measured. The results are shown in Table 3.

TABLE 3

| Human IgG (µg) | $(\Delta OD_{340nm}/min)$ |
|---|---|
| 0 | 0.080 |
| 5 | 0.070 |
| 25 | 0.062 |
| 50 | 0.050 |
| 200 | 0.035 |
| 800 | 0.028 |

The same measurements were then carried out on 4 serum samples, using in each case, 50 µl serum. The IgG concentration of each sample was determined by using the results of Table 3 plotted as a calibration curve. In a comparative test, the IgG concentrations of the same sera were also measured by the SRID method which is a conventional method.

The results obtained are shown in Table 4.

TABLE 4

| | IgG Concentration | |
|---|---|---|
| Serum | The Method of the Invention | SRID Method |
| B1 | 12.2 mg/ml | 11.8 mg/ml |
| B2 | 11.8 mg/ml | 12.0 mg/ml |
| B3 | 8.9 mg/ml | 8.2 mg/ml |
| B4 | 14.3 mg/ml | 13.5 mg/ml |

Example 3

i) Preparation of Anti-Human IgG Guinea-pig IgG-Antihexokinase Guinea-pig IgG bound complex

The preparation was carried out in the same manner as stage i) of Example 2 with the difference only that antihexokinase guinea-pig IgG (α-HKIgG) was employed instead of the α-G6PDHIgG, and a fraction containing 4 mg of α-hIgG-α-HKIgG combination was obtained.

ii) Measurement of Human IgG

50 l samples of the above α-hIgG-α-HKIgG bound complex were each added to 50 µl of human IgG solution having various known IgG concentrations. 100 l of 1/100 diluted solution of α-guinea-pig IgG-IgG serum were added to each mixture, and the mixtures obtained were kept at 25°C for 1 hour. 25 µl of a solution containing 0.4 g of hexokinase were then added to each mixture. Reaction at 25°C was allowed to take place for 30 minutes. 3 ml of 50 mM tris buffer solution of pH 8.0 containing 0.1M glucose, 0.5 mM ATP, 0.2 mM NADP, 3 U.ml glucose-6-phosphate dehydrogenase, and 13.3 mM $MgCl_2$ were then added as substrate to each mixture and the variation of absorbance at 340 nm at 25°C was measured. The results obtained are shown in Table 5.

8

TABLE 5

| Human IgG | $(\Delta\ OD_{340nm}/min) \times 1000$ |
|---|---|
| 0.6 μg/ml | 19.5 |
| 2.0 μg/ml | 17.0 |
| 5.0 μg/ml | 14.1 |
| 20.0 μg/ml | 11.3 |
| 100 μg/ml | 8.5 |

The same measurements were then carried out on five human serum samples, using in each case 50 μl of 1000 times diluted serum. The IgG concentration of each sample was determined by using the results of Table 5 plotted as a calibration curve. For comparative purposes, the IgG concentrations of the same sera were also measured by the conventional SRID method.

The results obtained are shown in Table 6.

TABLE 6

| | IgG Concentration | |
|---|---|---|
| Serum | The Method of the Invention | SRID Method |
| C1 | 8.4 mg/ml | 9.1 mg/ml |
| C2 | 16.1 mg/ml | 17.2 mg/ml |
| C3 | 11.5 mg/ml | 10.3 mg/ml |
| C4 | 12.1 mg/ml | 11.8 mg/ml |
| C5 | 7.5 mg/ml | 8.1 mg/ml |

Example 4

i) Preparation of Anti-Glucose-6-Phosphate Dehydrogenase Mouse IgG

G6PDP derived from a yeast (manufactured by Oriental Yeast Co. Ltd.) was employed as an antigen. A 1 mg/ml solution in water of this G6PDH was mixed with an equal volume of Freund complete adjuvant to form an emulsion, and 0.1 ml of this emulsion was injected three times into the abdominal cavity of a BALB/C mouse of 8 weeks growth every two weeks. After a further week, 50 μg/0.1 ml of G6PDH solution were injected into the tail vein of the mouse, and 3 days later, its spleen was excised. This spleen was ground, and spleen cell was separated. The spleen cell was fused with mouse myeloma P3U1 cell using polyethylene glycol 1500.

Hybridoma thus produced was placed in each well of a plate having 96 wells, and cultured in HAT medium. The cell content of each well was examined by the ELISA method using a plate on which G6PDH was immobilised, and 5 wells containing mouse IgG specific to G6PDH were found. The cells of these 5 wells were diluted by the limit dilution method and cloned, and two cell lines recognising different antigenic determinants of G6PDH were obtained by examining the results of the inhibition method test by the ELISA method.

Each cell line was multiplied in 10% FCS-RPMI medium, and $10^7$ of each multiplied cell line were injected into the abdominal cavity of a BALB/C mouse into which pristane had previously been injected. After two weeks, about 10 ml of ascites was withdrawn. The ascites were treated by the precipitation method using ammonium sulphate in 45% saturation, and the precipitates formed were separated. The precipitates were dissolved in a small amount of a phosphate buffer solution of pH 7.0, and separated by gel filtration using a Sephacryl® S—300 column which had previously been equilibrated with the same buffer solution. The IgG fractions were collected.

Equal amounts of two IgG samples thus obtained and recognising different antigenic determinants were mixed with each other, and used as the anti-glucose-6-phosphate dehydrogenase mouse IgG (α-G6PDHIgG).

ii) Preparation of Human α-Fetoprotein Mouse IgG

Following the same procedure as in stage i), two kinds of mouse monoclonal antibodies against AFP were prepared. Each antibody was purified to obtain IgG, and they were mixed with each other and used as human-fetoprotein mouse IgG (α-AFP IgG).

iii) Preparation of α-AFPIgG-α-G6PDHIgG Bound Complex

50 mg of dextran T500® (mean molecular weight: 500,000, manufactured by Pharmacia Fine Chemicals) were dissolved in 1 ml of water, and 0.2 ml of 0.1M sodium periodate was added. Then, the mixed solution was allowed to stand at 4°C overnight for reaction to take place. 0.15 ml of ethylene glycol was added to the reaction mixture, and allowed to react for 5 minutes. The reaction mixture was then treated by gel filtration using a Sephadex® G—25 column which had previously been equilibrated with 1 mM sodium acetate buffer solution of pH 5.0, and void fractions were collected. A solution of 20 mg of a mixture of α-AFPIgG and α-G6PDHIgG dissolved in 10 mM carbonate buffer solution of pH 9.5 was added to the said fractions, and the pH of this mixed solution was adjusted to 9.5. This mixed solution was then allowed to react at ambient temperature for 2 hours after which 0.5 ml of 0.4% sodium borohydride was added thereto, and further reaction was allowed to take place at 4°C for 2 hours. This reaction mixture was dialysed against 20 mM phosphate buffer solution of pH 7.0. The dialysate was separated by gel filtration using a Sephacryl® S—300 column, and the fractions of the α-AFPIgG-α-G6PDHIgG bound complex obtained were collected.

iv) Measurement of AFP

50 μl samples of the above fractions of α-AFPIgG-α-G6PDHIgG bound complex were each added to a 50 l sample of AFP having various known concentrations. A 100 μl sample of rabbit anti mouse IgG IgG fraction containing G6PDH was added to each sample of the mixture, and reaction was allowed to occur at 25°C for 30 minutes. 1.0 ml of 0.1M glycylglycine buffer solution of pH 8.5 containing 0.5 mM glucose-6-phosphate, 0.5 mM NADP and 20 mM $MgCl_2$ was added as the substrate for G6PDH, and the variation in rate of absorbance at 340 nm at 25°C for different AFP concentrations was measured. The results obtained are shown in Table 7.

TABLE 7

| AFP | $(\Delta\ OD_{340nm}/min) \times 1000$ |
|---|---|
| 0 ng | 36.5 |
| 50 ng | 28.1 |
| 100 ng | 22.3 |
| 200 ng | 19.8 |
| 400 ng | 16.9 |
| 800 ng | 15.1 |

The same measurements were carried out on five human serum samples, using 50 μl of each sample serum. The AFP concentration of each sample was determined by using the results of Table 7 plotted as a calibration curve. For comparative purposes the AFP concentrations of the same sera were also measured by the conventional RIA method.

The results obtained are shown in Table 8.

TABLE 8

AFP Concentration

| Serum | The Method of the Invention | RIA Method |
|-------|------------------------------|------------|
| D1 | 200 ng | 187 ng |
| D2 | 60 ng | 53 ng |
| D3 | 320 ng | 334 ng |
| D4 | 530 ng | 551 ng |
| D5 | 105 ng | 112 ng |

Example 5

i) Preparation of Theophylline-Dextran Bound Complex

1 g of dextran having a molecular weight of about 2,000,000 was suspended in 50 ml of 1N sodium hydroxide solution in 90% ethanol. 1 g of chloroacetic acid was added to this solution, and stirred at 37°C for 16 hours. The precipitate which formed was collected by filtration, washed sufficiently with ethanol, and dissolved in water. This aqueous solution was introduced into the column packed with Sephadex® G—25, and unreacted chloroacetic acid was removed. The carboxymethyl dextran fractions which were void fractions were collected, and lyophilised.

500 mg of this carboxymethyl dextran were suspended in dioxane, and 500 mg of N-hydroxysuccinimide and 500 mg of soluble carbodiimide were added. The mixture obtained was stirred overnight at ambient temperature. The precipitated material was collected on a glass filter and washed sufficiently with dioxane and then with diethyl ether. This washed precipitate of the succinimide ester of carboxymethyl dextran was then dried.

200 mg of this carboxymethyl dextran succinimide ester were added to 0.1M hexamethylenediamine solution of pH 8.0, and stirred at ambient temperature for 2 hours. Subsequently, gel filtration was carried out by using a column of Sephadex® G—25, and void fractions were collected. The fractions were lyophilised to yield lyophilised dextran induced amino group.

10 mg of 3-carboxytheophylline and 100 mg of the above dextran induced amino group were dissolved in water, and the pH of the solution was adjusted to 6.0. 20 mg of soluble carbodiimide were added to the solution, and reaction was allowed to take place for 1 hour while the pH was maintained at 6.0. The reaction mixture was then introduced into a column of Sephadex® G—25 which has previously been equilibrated with 20 mM phosphate buffered saline solution of pH 7.0, and gel filtration was carried out. Void fractions were collected, and lyophilised to yield the desired theophylline-dextran bound complex.

ii) Preparation of Antitheophylline Rabbit Antibody-Anti-G6PDH Mouse Monoclonal Antibody Bound Complex

The procedure of stage i) of Example 2 was repeated, but with the difference that antitheophylline rabbit IgG and anti-G6PDH mouse monoclonal antibody (anti-G6PDH antibody) were employed instead of α-hIgG and α-G6PDHIgG. The fraction containing antitheophylline rabbit antibody-anti-G6PDH antibody combination in a molar ratio of 1:1 was collected.

iii) Measurement of Theophylline

50 µl samples of a solution containing 30 µg of the theophylline-dextran bound complex and 100 µg of the aforesaid antibodies bound complex were each added to 50 µl of one of a number of theophylline solutions each having a known concentration, and kept at 37°C for 30 minutes. Then, 50 µl of a solution containing 1 µg of G6PDH were added to each mixed solution, and after 30 minutes, 1.0 ml of 0.1M glycyl-glycine buffer solution pH 8.5 containing 0.5 nM glucose-6-phosphoric acid, 0.5 mM NADP and 20 mM $MgCl_2$ was added. The variation of absorbance at 340 nm at 30°C was measured for each theophylline solution used. The results are shown in Table 9.

11

# EP 0 119 767 B1

TABLE 9

| Theophylline | $\Delta\ OD_{340nm}/min$ |
|---|---|
| 0 µg | 0.091 |
| 2.0 µg | 0.081 |
| 5.0 µg | 0.072 |
| 10.0 µg | 0.054 |
| 20.0 µg | 0.032 |
| 30.0 µg | 0.026 |
| 40.0 µg | 0.024 |

The same measurements were then carried out on five human serum samples using 50 l of each serum sample, and the theophylline concentration of each sample was determined by using the results of Table 9 plotted as a calibration curve. For comparison, the theophylline concentrations of the same sera were also measured by the conventional RIA method.

The results obtained are shown in Table 10.

TABLE 10

| Serum | Theophylline Concentration | |
|---|---|---|
| | The Method of the Invention | RIA Method |
| E1 | 0.5 µg/ml | 0.31 µg/ml |
| E2 | 2.0 µg/ml | 2.6 µg/ml |
| E3 | 15.0 µg/ml | 14.6 µg/ml |
| E4 | 13.1 µg/ml | 13.3 µg/ml |
| E5 | 10.6 µg/ml | 10.1 µg/ml |

## Example 6

i) Preparation of Anti-Human IgG Goat IgG F(ab')$_2$

10 mg of anti-human IgG goat IgG were dissolved in 2 ml of 0.1M sodium acetate solution of pH 4.2, and 100 µg of pepsin were added to the solution which was stirred at 37°C overnight. The pH of this digesting solution was adjusted to 7.5, and gel filtration was carried out by using Sephadex® G—100. The fractions of molecular weight about 100,000 were then collected, and concentrated by using polyethylene glycol. In this way, anti-human IgG goat IgG F(ab')$_2$ (containing 6 mg of protein) was obtained.

ii) Preparation of Anti-G6PDH Mouse IgG Fab

10 mg of anti G6PDH mouse IgG obtained in step i) of Example 4 were treated in the same manner as in stage i), and 3.6 mg of anti-G6PDH mouse IgG Fab were obtained.

iii) Preparation of Macromolecular Human IgG

5 mg of human IgG were dissolved in 1 ml of 10 mM phosphate buffer solution of pH 6.0, and 10 mg of soluble carbodiimide were added. This solution was kept at pH 6.0 by using 0.1M(N) NaOH and 0.1M(N) HCl, and when the solution became somewhat turbid, the solution was passed through a column of Sephadex® G—25 which had previously been equilibrated with 20 mM phosphate buffer solution of pH 7.0 and deionised. Void fractions were collected, and further treated by gel filtration using Sepharose® 4B, and void fractions were collected to obtain the macromolecular human IgG.

iv) Preparation of Anti-Human IgG Goat IgG Fab-Anti-G6PDH Mouse IgG Fab Bound Complex

2 mg of anti-G6PDH mouse IgH Fab were dissolved in 1 ml of 0.1M phosphate buffer solution of pH 6.0, and 100 µl of 2.0 mg/ml CHMS acetone solution were added, and allowed to react at 30°C for 90 minutes. The reaction mixture then obtained was introduced into a Sephadex® G—25 column which had previously been equilibrated with 0.1M phosphate buffer solution of pH 6.3, and gel filtration was carried out. Void fractions were collected, and concentrated to 1 ml to obtain 2 mg of CHM induced anti-G6PDH mouse IgG Fab.

1 mg of anti-human IgG goat IgG F(ab')$_2$ was then dissolved in 0.1M phosphate buffer solution of pH 6.0, and 100 µl of 56 mg/ml distilled water of 2-mercaptoethylamine solution were added. The mixed solution obtained was allowed to react at 37°C for 1.5 h. The reaction mixture then obtained was treated by gel filtration using Sephadex® G—25 which had previously been equilibrated using 0.1M phosphate buffer solution of pH 6.3 containing 1 mM EDTA. Void fractions were collected, and concentrated to 1 ml by using polyethylene glycol.

This concentrate was mixed with the above CHM induced anti-G6PDH mouse IgG Fab solution, and allowed to stand overnight at 4°C. Subsequently, gel filtration was carried out by using Sephadex® G—150 which had previously been equilibrated with 20 mM phosphate buffered saline solution of pH 7.0, and the fractions of molecular weight of about 100,000 were collected. The fractions were concentrated to 2 ml, to yield the target bound complex.

v) Measurement of Human IgG

50 µl samples of a solution containing 1.0 mg of the macromolecular human IgG were placed in small test tubes, and 50 µl of a solution containing human IgG on one of the concentrations described in Table 11 and 50 µl of the above concentrate of anti-G6DPH mouse IgG Fab-anti-human IgG goat IgG Fab bound complex were added to each test tube. The mixed solutions obtained were allowed to stand at 37°C for 30 minutes. 50 µl of a solution containing 1 µg of G6PDH were added to each and the test tubes were allowed to stand at 37°C for 30 minutes. Subsequently, 1.0 ml of a substrate solution of pH 8.5 containing 0.5 mM glucose-6-phosphoric acid, 0.15 mM NADP$^+$, 20 mM MgCl$_2$ and 0.1M glycylcycline was added and the variation of absorbance at 340 nm with human IgG concentration was measured. The results obtained are shown in Table 11.

TABLE 11

| Human IgG | $\Delta$ OD$_{340nm}$/min |
|-----------|---------------------------|
| 0 µg | 0.090 |
| 100 µg | 0.080 |
| 300 µg | 0.062 |
| 600 µg | 0.041 |
| 1200 µg | 0.032 |
| 2500 µg | 0.025 |

The same measurements were then carried out on five human serum samples using 50 µl of each serum sample, and the IgG concentration of each sample was determined by using the results of Table 11 plotted as a calibration curve. For comparative purposes on the other hand, the IgG concentrations of the same sera were also measured by the conventional SRID method.

The results obtained are shown in Table 12.

EP 0 119 767 B1

TABLE 12

IgG Concentration

| Serum | The Method of the Invention | SRID Method |
|---|---|---|
| F1 | 11.2 mg/ml | 10.9 mg/ml |
| F2 | 8.6 mg/ml | 9.1 mg/ml |
| F3 | 13.5 mg/ml | 12.1 mg/ml |
| F4 | 13.7 mg/ml | 13.6 mg/ml |
| F5 | 12.2 mg/ml | 11.8 mg/ml |

**Claims**

1. A method of determining a biological ligand which comprises, bringing about contact in an aqueous medium between substances (a) to (c) of which substance (a) is a sample considered to contain a ligand to be measured, the ligand being of macromolecular size or a hapten, substance (b) is an enzyme or a bound complex of an enzyme and a water-soluble macromolecular substance, and substance (c) is either a bound complex of an antibody against said ligand and an antibody against said enzyme or a bound complex of an antibody against said ligand, and antibody against said enzyme and a water-soluble macromolecular substance, one of substances (a), (b) and (c) being of macromolecular size with a substance (b) or (c), in the absence of a substance (a) of macromolecular size, achieving such macromolecular size as a result of it being a said bound complex with a water-soluble macromolecular substance having a molecular weight greater than 100,000; determining the residual activity of said enzyme and utilising the result obtained as a measure of the ligand or an indication of the absence thereof.

2. A method as claimed in claim 1, wherein said substance (a) and said substance (b) are brought into contact in the aqueous medium and the mixture obtained is contacted with said substance (c).

3. A method as claimed in claim 1 wherein, there is additionally introduced into said aqueous solution a further substance (d) which is a bound complex of said ligand and a water-soluble macromolecular substance and/or polymerised form of said ligand.

4. A method of determining a biological ligand which comprises bringing about contact in an aqueous medium between substances (a) to (d) of which substance (a) is a sample considered to contain the ligand to be measured, the ligand being of macromolecular size or a hapten, substance (b) is an enzyme or a bound complex of an enzyme and a water-soluble macromolecular substance, substance (c) is either a bound complex of an antibody against said ligand and an antibody against said enzyme or a bound complex of an antibody against said ligand, an antibody against said enzyme and a water-soluble macromolecular substance and substance (d) is a bound complex of a said ligand and a water-soluble macromolecular substance which has a molecular weight of more than 100,000 daltons and/or a polymerized form of said ligand; determining the residual activity of said enzyme and utilising the result obtained as a measure of the ligand or an indication of the absence thereof.

5. A method as claimed in claim 3 or 4, wherein said substance (a) and enzyme or bound complex (b) are brought into contact in the aqueous medium and the mixture obtained is contacted with said bound complex (c) and said bound complex and/or polymerised ligand (d).

6. A method of determining a biological ligand which comprises bringing about contact in an aqueous medium between substances (a) to (c) and (e) of which substance (a) is a sample considered to contain the ligand to be measured, the ligand being of macromolecular size or a hapten, substance (b) is an enzyme or a bound complex of an enzyme and a water-soluble macromolecular substance, substance (c) is either a bound complex of an antibody against said ligand and an antibody against said enzyme or a bound complex of an antibody against said ligand, an antibody against the enzyme and a water-soluble macromolecular substance and substance (e) is a second antibody which is an antibody against said antibody against said ligand or a bound complex of a said second antibody against said antibody against said ligand and a water-soluble macromolecular substance and/or is a second antibody which is an antibody against said antibody against said enzyme or a bound complex of a said antibody against said antibody against said enzyme and a water-soluble macromolecular substance; determining the residual activity of said enzyme and utilising the result obtained as a measure of the ligand or an indication of the absence thereof.

7. A method as claimed in claim 6, wherein a said water-soluble macromolecular substance has a molecular weight of more than 100,000 daltons.

8. A method as claimed in claim 6 or 7, wherein said substance (a) and said substance (b) are brought

14

# EP 0 119 767 B1

into contact in the aqueous medium and the mixture obtained is contacted with said substance (c) and said substance(s) (e).

9. A method as claimed in any one of claims 1 to 8, wherin said ligand has a molecular weight of more than 100,000 daltons.

10. A method as claimed in any one of claims 1 to 8, wherein a said ligand of macromolecular size is a hormone derived from an endocrine gland, a plasma protein, a viral antigen, a bacterium or a carcinoembryonic antigen.

11. A method as claimed in any one of claims 1 to 8, wherein said ligand has a molecular weight of less than 100,000 daltons.

12. A method as claimed in claim 11, wherein said ligand is a hapten which is a medicinal substance or a hormone.

13. A method as claimed in any one of the preceding claims, wherein said enzyme is glucose-6-phosphate dehydrogenase, hexokinase, α-amylase, malate dehydrogenase, alkaline phosphatase, peroxidase, β-galactosidase, creatinekinase, ribonuclease, or pencillinase.

14. A method as claimed in any preceding claim, wherein a said macromolecular substance is a polysaccharide or derivative thereof, a protein or polyethylene glycol.

15. A method as claimed in claim 14, wherein said polysaccharide or derivative thereof is a water-soluble dextran, carboxymethyl dextran, a dextran induced amino group or amylose, and said protein is gelatin, hemocyanin or ferritin.

16. A method as claimed in any one of the preceding claims, wherein one or more said antibodies introduced into the aqueous solution is/are a monoclonal antibody(ies).

17. A method as claimed in any one of claims 1 to 15, wherein one or more said antibodies introduced into said aqueous solution is/are a F(ab')$_2$, Fab' or FAB digestion product of the antibody concerned.

## Patentansprüche

1. Verfahren zur Bestimmung eines biologischen Liganden, das besteht aus:
Zustandebringen eines Kontaktes in einem wässrigen Medium zwischen Substanzen (a) bis (c), bei denen

Substanz (a) eine Probe ist, die einen zu messenden Liganden beinhalten dürfte, der Ligand von makromolekulare Größe oder ein Hapten ist,

Substanz (b) ein Enzym oder ein gebundener Komplex eines Enzyms und einer wasserlöslichen makromolekularen Substanz ist und

Substanz (c) entweder ein gebundener Komplex eines Antikörpers gegen den Liganden und eines Antikörpers gegen das Enzym oder ein gebundener Komplex eines Antikörpers gegen den Liganden, eines Antikörpers gegen das Enzym und einer wasserlöslichen makromolekularen Substanz ist,

eine von den Substanzen (a), (b) oder (c) von makromolekularer Größe ist,

wobei eine Substanz (b) oder (c), in Abwesenheit einer Substanz (a) von makromolekularer Größe, diese makromolekulare Größe erreicht, weil sie ein gebundener Komplex mit einer wasserlöslichen makromolekularen Substanz ist, die ein Molekulargewicht von größer als 100.000 besitzt;

Bestimmen der Restaktivität des Enzyms und

Verwenden des erhaltenen Ergebnisses als eine Messung des Liganden oder als einen Hinweis auf dessen Abwesenheit.

2. Verfahren nach Anspruch 1, bei dem Substanz (a) und Substanz (b) in dem wässrigen Medium in Kontakt gebracht werden und die erhaltene Mischung mit Substanz (c) in Kontakt gebracht wird.

3. Verfahren nach Anspruch 1, bei dem zusätzlich in die wässrige Lösung eine weitere Substanz (d) eingebracht wird, die ein gebundener Komplex des Liganden und einer wasserlöslichen makromolekularen Substanz und/oder einer polymerisierten Form des Liganden ist.

4. Verfahren zur Bestimmung eines biologischen Liganden, das besteht aus:
Zustandebringen eines Kontaktes in einem wässrigen Medium zwischen Substanzen (a) bis (d), bei denen

Substanz (a) eine Probe ist, die einen zu messenden Liganden beinhalten dürfte, wobei, der Ligand von makromolekulare Größe oder ein Hapten ist,

Substanz (b) ein Enzym oder ein gebundener Komplex eines Enzyms und einer wasserlöslichen makromolekularen Substanz ist,

Substanz (c) entweder ein gebundener Komplex eines Antikörpers gegen den Liganden und eines Antikörpers gegen das Enzym oder ein gebundener Komplex des Antikörpers gegen den Liganden, eines Antikörpers gegen das Enzym und einer wasserlöslichen makromolekularen Substanz ist und

Substanz (d) ein gebundener Komplex des Liganden und einer wasserlöslichen makromolekularen Substanz, die ein

Molekulargewicht von mehr als 100.00 Dalton besitzt, und/oder einer polymerisierten Form des Liganden ist;

Bestimmen der Restaktivität des Enzyms und

Verwenden des erhaltenen Ergebnisses als eine Messung des Liganden oder als einen Hinweis auf dessen Abwesenheit.

15

5. Verfahren nach Anspruch 3 oder 4, bei dem Substanz (a) und Enzym oder gebundener Komplex (b) in dem wässrigen Medium in Kontakt gebracht werden und die erhaltene Mischung mit dem gebundenen Komplex (c) und dem gebundenem Komplex und/oder polymerisiertem Liganden (d) in Kontakt gebracht wird.

6. Verfahren zur Bestimmung eines biologischen Liganden, das besteht aus:

Zustandebringen eines Kontaktes in einem wässrigen Medium zwischen den Substanzen (a) bis (c) und (e), bei denen

Substanz (a) eine Probe ist, die einen zu messenden Liganden beinhalten dürfte, wobei, der Ligand von makromolekulare Größe oder ein Hapten ist,

Substanz (b) ein Enzym oder ein gebundener Komplex eines Enzyms und einer wasserlöslichen makromolekularen Substanz ist,

Substanz (c) entweder ein gebundener Komplex eines Antikörpers gegen den Liganden und eines Antikörpers gegen das Enzym oder ein gebundener Komplex des Antikörpers gegen den Liganden, eines Antikörpers gegen das Enzym und einer wasserlöslichen makromolekularen Substanz ist,

Substanz (e) ein zweiter Antikörper, der ein Antikörper gegen den Antikörper gegen den Liganden ist oder ein gebundener Komplex des zweiten Antikörpers gegen den Antikörper gegen den Liganden und einer wasserlöslichen makromolekularen Substanz ist und/oder ein zweiter Antikörper, der ein Antikörper gegen den Antikörper gegen das Enzym ist, oder ein gebundener Komplex des Antikörpers gegen den Antikörper gegen das Enzym und einer wasserlöslichen makromolekularen Substanz ist;

Bestimmen der Restaktivität des Enzyms und

Verwenden des erhaltenen Ergebnisses als eine Messung des Liganden oder als einen Hinweis auf dessen Abwesenheit.

7. Verfahren nach Anspruch 6, bei dem die wasserlösliche makromolekulare Substanz ein Molekulargewicht von mehr als 100.000 Dalton besitzt.

8. Verfahren nach Anspruch 6 oder 7, wobei Substanz (a) und Substanz (b) in dem wässrigen Medium in Kontakt gebracht werden und die erhaltene Mischung mit Substanz (c) und Substanz(en) (e) in Verbindung gebracht wird.

9. Verfahren nach einem der vorhergehenden Ansprüche 1 bis 8, bei dem der Ligand ein Molekulargewicht von mehr als 100.000 Dalton besitzt.

10. Verfahren nach einem der vorhergehenden Ansprüche 1 bis 8, bei dem der Ligand mit makromolekularer Größe ein Hormon ist, hergeleitet von einer endokrinen Drüse, einem Plasmaprotein, einem viralen Antigen, einem Bakterium oder einem karcinogen embryonischen Antigen.

11. Verfahren nach einem der vorhergehenden Ansprüche 1 bis 8, bei dem der Ligand ein Molekulargewicht von weniger als 100.000 Dalton besitzt.

12. Verfahren nach Anspruch 11, bei dem der Ligand ein Hapten ist, das eine medizinische Substanz oder ein Hormon ist.

13. Verfahren nach einem der vorhergehenden Ansprüche, bei dem das Enzym Glucose-6-Phosphatdehydrogenase, Hexokinase, α-Amylase, Malat-Dehydrogenase, alkalische Phosphatase, Peroxidase, β-Galactosidase, Kreatinkinase, Ribonuclease oder Penicillinase ist.

14. Verfahren nach einem der vorhergehenden Ansprüche, bei dem die makromolekulare Substanz ein Polysaccharid oder ein Derivat davon, ein Protein oder Polyethylenglycol ist.

15. Verfahren nach Anspruch 14, bei dem das Polysaccharid oder das Derivat davon ein wasserlösliches Dextran, Carboxymethyldextran, eine dextraninduzierte Aminogruppe oder Amylose ist und das Protein Gelantine, Hämocyanin oder Ferritin ist.

16. Verfahren nach einem der vorhergehenden Ansprüche, bei dem einer oder mehrere Antikörper, die in die wässrige Lösung einbracht werden, ein/mehrere monoklonale Antikörper ist/sind.

17. Verfahren nach einem der vorhergehenden Ansprüche 1 bis 15, bei dem einer oder mehrere Antikörper, die in die wässrige Lösung eingebracht werden, ein $F(ab')_2$, Fab' oder FAB Abbauprodukt des betrachteten Antikörpers ist bzw. sind.

## Revendications

Procédé pour la détermination d'un ligand biologique, lequel comprend la mise en contact, dans un milieu aqueux, des substances (a) à (c), dont la substance (a) est un échantillon considéré comme contenant un ligand à doser, le ligand étant d'une taille macromoléculaire ou un haptène, la substance (b) est une enzyme ou un complexe lié d'une enzyme et d'une substance macromoléculaire soluble dans l'eau, et la substance (c) est soit un complexe lié d'un anticorps dirigé contre ledit ligand et d'un anticorps dirigé contre ladite enzyme, soit un complexe lié d'un anticorps dirigé contre ledit ligand, d'un anticorps dirigé contre ladite enzyme et d'une substance macromoléculaire soluble dans l'eau, l'une des substances (a), (b) et (c) étant de taille macromoléculaire, avec une substance (b) ou (c) atteignant, en l'absence d'une substance (a) de taille macromoléculaire, cette taille macromoléculaire du fait qu'elle est un dit complexe lié avec une substance macromoléculaire soluble dans l'eau, ayant une masse moléculaire supérieure à 100 000; la détermination de l'activité résiduelle de ladite enzyme et l'utilisation du résultat obtenu comme mesure du ligand ou comme une indication de l'absence de celui-ci.

2. Procédé selon la revendication 1, dans lequel ladite substance (a) et ladite substance (b) sont mises en contact dans le milieu aqueux et le mélange obtenu est mis en contact avec ladite substance (c).

3. Procédé selon la revendication 1, dans lequel on introduit en outre dans ladite solution aqueuse une autre substance (d) qui est un complexe lié dudit ligand et d'une substance macromoléculaire soluble dans l'eau et/ou une forme polymérisée dudit ligand.

4. Procédé pour la détermination d'un ligand biologique, lequel comprend la mise en contact, dans un milieu aqueux, des substances (a) à (d), dont la substance (a) est un échantillon considéré comme contenant le ligand à doser, le ligand étant de taille macromoléculaire ou un haptène, la substance (b) est une enzyme ou un complexe lié d'une enzyme et d'une substance macromoléculaire soluble dans l'eau, la substance (c) est soit un complexe lié d'un anticorps dirigé contre ledit ligand et d'un anticorps dirigé contre ladite enzyme, soit un complexe lié d'un anticorps dirigé contre ledit ligand, d'un anticorps dirigé contre ladite enzyme et d'une substance macromoléculaire soluble dans l'eau, et la substance (d) est un complexe lié d'un dit ligand et d'une substance macromoléculaire soluble dans l'eau, ayant une masse moléculaire de plus de 100 000 daltons et/ou une forme polymérisée dudit ligand; la détermination de l'activité résiduelle de ladite enzyme et l'utilisation du résultat obtenu comme mesure du ligand ou comme une indication de l'absence de celui-ci.

5. Procédé selon la revendication 3 ou 4, dans lequel ladite substance (a) et l' enzyme ou le complexe lié (b) sont mis en contact dans le milieu aqueux, et le mélange obtenu est mis en contact avec ledit complexe lié (c) et ledit complexe lié et/ou ligand polymérisé (d).

6. Procédé pour la détermination d'un ligand biologique, lequel comprend la mise en contact, dans un milieu aqueux, des substances (a) à (c) et (e), dont la substance (a) est un échantillon considéré comme contenant le ligand à doser, le ligand étant de taille macromoléculaire ou un haptène, la substance (b) est une enzyme ou un complexe lié d'une enzyme et d'une substance macromoléculaire soluble dans l'eau, la substance (c) est soit un complexe lié d'un anticorps dirigé contre ledit ligand et d'un anticorps dirigé contre ladite enzyme, soit un complexe lié d'un anticorps dirigé contre ledit ligand, d'un anticorps dirigé contre ladite enzyme et d'une substance macromoléculaire soluble dans l'eau, et la substance (e) est un second anticorps qui est un anticorps dirigé contre ledit anticorps dirigé contre ledit ligand ou un complexe lié d'un dit second anticorps dirigé contre ledit anticorps dirigé contre ledit ligand et d'une substance macromoléculaire soluble dans l'eau et/ou est un second anticorps qui est un anticorps dirigé contre ledit anticorps dirigé contre ladite enzyme, ou un complexe lié d'un dit anticorps dirigé contre ledit anticorps dirigé contre ladite enzyme, et d'une substance macromoléculaire soluble dans l'eau; la détermination de l'activité résiduelle de ladite enzyme et l'utilisation du résultat obtenu comme mesure du ligand ou comme une indication de l'absence de celui-ci.

7. Procédé selon la revendication 6, dans lequel une dite substance macromoléculaire soluble dans l'eau a une masse moléculaire de plus de 100 000 daltons.

8. Procédé selon la revendication 6 ou 7, dans lequel ladite substance (a) et ladite substance (b) sont mises en contact dans le milieu aqueux, et le mélange obtenu est mis en contact avec ladite substance (c) et ladite(lesdites) substance(s) (e).

9. Procédé selon l'une quelconque des revendications 1 à 8, dans lequel ledit ligand a une masse moléculaire de plus de 100 000 daltons.

10. Procédé selon l'une quelconque des revendications 1 à 8, dans lequel un dit ligand de taille macromoléculaire est une hormone provenant d'une glande endocrine, une protéine plasmatique, un antigène viral, une bactérie ou un antigène carcino-embryonnaire.

11. Procédé selon l'une quelconque des revendications 1 à 8, dans lequel ledit ligand a une masse moléculaire de moins de 100 000 daltons.

12. Procédé selon la revendication 11, dans lequel ledit ligand est un haptène qui est une substance médicinale ou une hormone.

13. Procédé selon l'une quelconque des revendications précédentes, dans lequel ladite enzyme est la glucose-6-phosphate déshydrogénase, l'hexokinase, l'α-amylase, la malate déshydrogénase, la phosphatase alcaline, la peroxydase, la β-galactosidase, la créatine kinase, la ribonucléase ou la pénicillinase.

4. Procédé selon l'une quelconque des revendications précédentes, dans lequel une dite substance macromoléculaire est un polysaccharide ou un dérivé de celui-ci, une protéine ou un polyéthylèneglycol.

15. Procédé selon la revendication 14, dans lequel ledit polysaccharide ou dérivé de celui-ci est un dextrane soluble dans l'eau, le carboxyméthyl-dextrane, un dextrane à groupe amino induit ou l'amylose, et ladite protéine est la gélatine, l'hémocyanine ou la ferritine.

16. Procédé selon l'une quelconque des revendications précédentes, dans lequel un ou plusieurs desdits anticorps introduits dans la solution aqueuse est/sont un(des) anticorps monoclonal(aux).

17. Procédé selon l'une quelconque des revendications 1 à 15, dans lequel un ou plusieurs desdits anticorps introduits dans ladite solution aqueuse est/sont un produit de digestion F(ab'$_2$), Fab' ou FAB de l'anticorps concerné.